# FASCICULE DE BREVET EUROPEEN

(11) **EP 3 003 141 B1**
(45) Date de publication et mention de la délivrance du brevet: **30.08.2023**
(21) Numéro de dépôt: 14728229.7
(22) Date de dépôt: 06.06.2014
(51) Int. Cl.: A61B 5/0507, A61B 5/00, G01S 13/89

(54) **SYSTÈME D'IMAGERIE MÉDICALE À ÉMISSION/RÉCEPTION MICRO-ONDES**
MEDIZINISCHES BILDGEBUNGSSYSTEM MIT MIKROWELLENSTRAHLUNG/-EMPFANG
MEDICAL IMAGING SYSTEM HAVING MICROWAVE EMISSION/RECEPTION

(30) Priorité: 06.06.2013 FR 1355225
(43) Date de publication de la demande: 13.04.2016
(73) Titulaire: MVG Industries, 91140 Villejust (FR); Office National d'Etudes et de Recherches Aérospatiales (ONERA), 91120 Palaiseau (FR)
(72) Inventeur: CASTELLI, Juan Carlos, 91120 Palaiseau (FR); DUCHESNE, Luc, 91470 Angervilliers (FR); TESSIER, Virginie, 92350 Le Plessis Robinson (FR); DURAND, Ludovic, 91460 Marcoussis (FR); GARREAU, Philippe, 75011 Paris (FR); ADNET, Nicolas, 91140 Villejust (FR); THIÉBAUT, Stéphane, 91140 Villejust (FR)
(74) Mandataire: Regimbeau
(86) Numéro de dépôt international: PCT/EP2014/061913
(87) Numéro de publication internationale: WO 2014/195502

(56) Documents cités:
- WO-A1-2005/078462
- WO-A1-2014/045181
- US-A1- 2004 077 943
- US-A1- 2005 107 692
- US-A1- 2006 241 409
- US-A1- 2009 015 832
- US-A1- 2013 018 591
- US-A1- 2013 116 572
- MACIEJ KLEMM ET AL: "Microwave Radar-Based Differential Breast Cancer Imaging: Imaging in Homogeneous Breast Phantoms and Low Contrast Scenarios", IEEE TRANSACTIONS ON ANTENNAS AND PROPAGATION, IEEE SERVICE CENTER, PISCATAWAY, NJ, US, vol. 58, no. 7, 1 juillet 2010 (2010-07-01), pages 2337-2344, XP011307911, ISSN: 0018-926X
- AMINEH R K ET AL: "Ultra-wide band TEM horn antenna for microwave imaging of the breast", ANTENNAS AND PROPAGATION SOCIETY INTERNATIONAL SYMPOSIUM, 2009. APSURSI '09. IEEE, IEEE, PISCATAWAY, NJ, USA, 1 juin 2009 (2009-06-01), pages 1-4, XP031536039, ISBN: 978-1-4244-3647-7
- FEAR E C ET AL: "Microwave System for Breast Tumor Detection", IEEE MICROWAVE AND GUIDED WAVE LETTERS, IEEE INC, NEW YORK, US, vol. 9, no. 11, 1 novembre 1999 (1999-11-01), XP011035499, ISSN: 1051-8207
- LIU CHAO ET AL: "A millimeter wave breast cancer imaging methodology", PRECISION ELECTROMAGNETIC MEASUREMENTS (CPEM), 2012 CONFERENCE ON, IEEE, 1 juillet 2012 (2012-07-01), pages 74-75, XP032210932, DOI: 10.1109/CPEM.2012.6250667 ISBN: 978-1-4673-0439-9

## Description

### DOMAINE GENERAL ET ART ANTERIEUR

La présente invention est relative à un système d'imagerie médicale à antennes micro-ondes.

Plus particulièrement, l'invention concerne des systèmes d'imagerie de ce type pour l'analyse de tissus ou d'organes humains perméables aux ondes électromagnétiques.

Elle trouve en particulier avantageusement application dans l'imagerie du sein.

Dans les systèmes d'imagerie micro-ondes, le choix du matériau de transition entre les antennes et le milieu sous observation est important afin d'assurer une bonne pénétration des ondes dans ce milieu. Les caractéristiques diélectriques du matériau de transition doivent être sélectionnées en fonction du milieu à observer, de façon à obtenir de meilleurs résultats au niveau des images.

De plus, idéalement, ce matériau de transition doit présenter le moins de pertes diélectriques possibles afin de limiter l'absorption de l'énergie de l'onde électromagnétique.

On prévoit le plus souvent à cet effet d'immerger les antennes dans un liquide avec les tissus biologiques à imager. Toutefois, même s'il est possible de modifier la nature du liquide afin d'obtenir la permittivité diélectrique voulue, un tel liquide présente généralement beaucoup de pertes diélectriques aux fréquences de travail souhaitées.

Il pose en outre des difficultés en termes d'étanchéité, d'isolation des sondes émetteurs/capteurs avec le milieu sous observation, d'hygiène, de nettoyage, de conservation dans le temps, de variation des caractéristiques électromagnétiques dans le temps et en fonction de la température.

Des systèmes d'imagerie micro-ondes employant des milieux de transition mixtes, comprenant un milieu de transition liquide et un milieu de transition solides ont également été proposés.

À titre d'exemple, l'article Microwave radar-based differential breast cancer imaging: imaging in homogeneous breast phantoms and low contrast scénarios - Klemm et al., IEEE - 2010 propose un système d'imagerie micro-ondes utilisant un milieu de transition solide consistant en une **cuve** en matériau céramique dans laquelle sont positionnées des coques de réglage adaptées aux différentes tailles de sein et en des antennes mécaniquement supportées par une enveloppe en matériau plastique qui est rapportée sur ladite cuve avec un milieu de transition liquide entre l'enveloppe en matériau plastique et la cuve en matériau céramique, ce milieu de transition liquide se trouvant entre les antennes et la **cuve.**

Un tel système pose toutefois un certain nombre de difficultés.

La structure d'émission/réception proposée dans l'article mentionné ci-dessus est limitée en termes de robustesse des résultats d'imagerie obtenus étant donné le faible nombre d'antennes fixes réparties autour de la coque.

Par ailleurs, l'utilisation d'une enveloppe en matériau plastique d'un côté et d'une coque en céramique de l'autre est une source d'échos pour les signaux au niveau de la transition entre les deux matériaux. Les mesures sont donc potentiellement perturbées.

Également, du fait de leur très grande proximité, les antennes d'émission et de réception ne sont pas parfaitement isolées électromagnétiquement les unes des autres. Un couplage électromagnétique existe entre elles, qui est d'autant plus important que les sondes sont proches. Or, le signal reçu par la sonde de réception et dû au couplage direct avec une sonde émettrice est généralement fort et limite la dynamique du récepteur. Pour reconstruire l'image du milieu observé, il est alors nécessaire de procéder à des traitements de soustraction des signaux. Les erreurs engendrées par ces soustractions sont d'autant plus importantes que le couplage direct est lui-même important.

Par ailleurs encore, la structure décrite dans cet article est nécessairement d'un câblage complexe et onéreux. Elle nécessite des matrices de commutateurs (et les branchements associés) particulièrement compliqués entre sondes. Cette structure nécessite soit des commutateurs pour sélectionner la sonde d'émission et la sonde de réception, soit des émetteurs et des récepteurs multiples ou la combinaison des deux. US2009/015832A1 traite également d'imagerie microonde de tissus, plus particulièrement du sein, utilisant des réseaux d'antennes d'émission et de réception et un design type cuve, coque et supports d'antennes.

### PRÉSENTATION DE L'INVENTION

L'invention qui est définie par l'objet des revendications 1 et 13, propose quant à elle une solution qui permet de pallier les inconvénients des techniques antérieures.

Notamment, la revendication 1 propose un système d'imagerie médicale à antennes d'émission et antennes de réception du champ électromagnétique disposées autour d'un volume destiné à recevoir un milieu de tissu humain à observer.

Il comporte un réseau d'antennes d'émission et un réseau d'antennes de réception, ces deux réseaux étant indépendants (et notamment mécaniquement indépendants). Des motorisations sont prévues qui sont adaptés pour déplacer le réseau d'émission et ou le réseau de réception en rotation angulaire et en translation par rapport au volume sous observation, afin de permettre un balayage de celui-ci. Le système est ainsi apte à fonctionner en multi-statisme total en termes d'imagerie.

Selon un aspect possible, les deux réseaux sont indépendants en rotation et en translation.

En ce qui concerne en particulier la translation, ceci permet un suréchantillonnage le long du déplacement en translation.

Avantageusement, le système comporte une coque en matériau diélectrique solide définissant le volume destiné à recevoir un milieu de tissu humain à observer, les antennes d'émission et les antennes de réception étant disposées en réseau dans des supports, la coque étant disposée dans une cuve en un matériau diélectrique que les réseaux entourent au moins partiellement, les matériaux de la cuve, de la coque et des supports de réseaux possédant des permittivités diélectriques identiques.

De préférence, lesdits supports comportent des parties en un matériau électromagnétiquement absorbant entre les antennes ou bien lesdits supports sont constitués entièrement du matériau électromagnétiquement absorbant. Ce matériau électromagnétiquement absorbant possède en outre une permittivité diélectrique identique à celle de la coque.

Le système comprend également avantageusement une électronique qui met en oeuvre une différenciation au moins des antennes de réception par modulation.

L'invention concerne également un procédé d'imagerie médicale d'un milieu de tissu humain à observer dans un système tel que défini par la revendication 1, et comprenant l'étape consistant à déplacer au moins l'un des réseaux d'émission et/ ou de réception par rapport au volume sous observation, afin de permettre un balayage de celui-ci, les micro-ondes émises par le réseau d'émission, et arrivant à une coque destinée à recevoir le milieu de tissu humain, traversant un milieu de transition uniquement solide.

### PRÉSENTATION DES FIGURES

D'autres caractéristiques et avantages de l'invention définie par les revendications ressortiront encore de la description qui suit, laquelle est purement illustrative et non limitative, et doit être lue en regard des figures annexées sur lesquelles :
- la figure 1 est une représentation schématique en demi-perspective illustrant un mode de réalisation possible pour l'invention ;
- la figure 2 est une représentation schématique en perspective d'un anneau d'antennes d'émission et d'un anneau d'antennes de réception du dispositif illustré sur la figure 1 ;
- la figure 3 illustre un autre mode de réalisation encore possible pour l'invention ; et
- la figure 4 illustre encore un autre mode de réalisation possible pour l'invention avec plusieurs réseaux d'émission et/ou de réception.

### DESCRIPTION D'UN OU PLUSIEURS MODES DE MISE EN ŒUVRE ET DE RÉALISATION

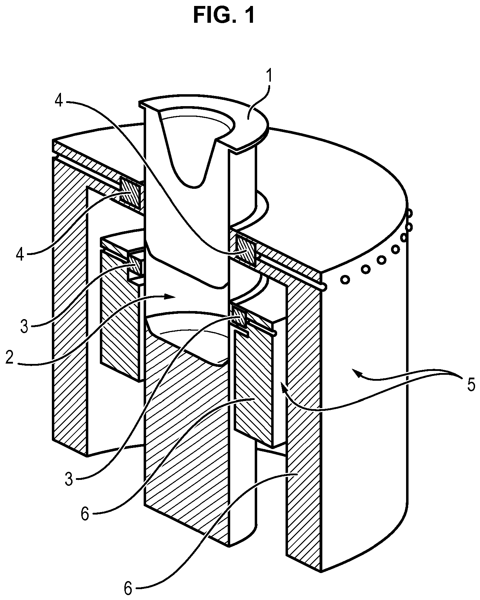

Dans le mode de réalisation de la figure 1, le dispositif comporte une coque en matériau diélectrique 1 destinée à recevoir le sein de la patiente, une cuve 2 dans laquelle est placée cette coque 1, au moins un réseau 3 de sondes d'émission 3a, au moins un réseau 4 de sondes de réception 4a, ainsi qu'un ensemble de carters métalliques 5 et d'absorbants électromagnétiques en mousse 6 qui entourent la cuve 2 et les réseaux 3 et 4.

La coque 1 a une forme extérieure complémentaire de la forme intérieure, en l'occurrence cylindrique, de la cuve 2 dans laquelle elle est maintenue. Elle présente en son intérieur une empreinte en creux destinée à recevoir le sein d'une patiente.

Cette coque 1 est amovible par rapport à la cuve 2. Le dispositif est associé à un jeu de coques 1. Les formes extérieures des coques du jeu de coques sont identiques. Les empreintes en revanche sont différentes et permettent l'adaptation à la taille des seins à imager.

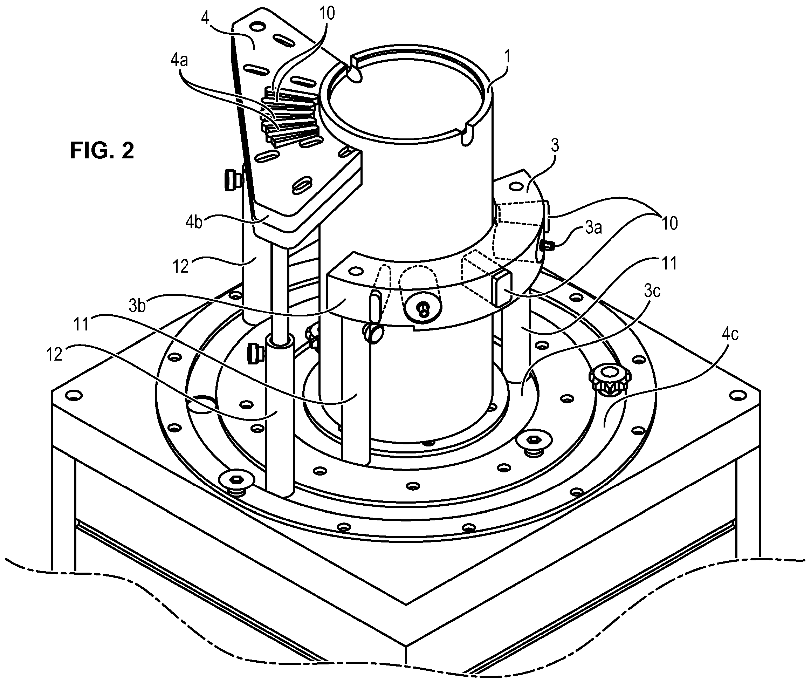

Le réseau 3 d'antennes d'émission 3a permet d'illuminer le milieu sous observation. En l'occurrence, les antennes 3a qui le composent peuvent être régulièrement réparties, de préférence dans un plan, de manière à entourer au moins partiellement la cuve 2 (figure 2), voire selon un anneau qui entoure la cuve 2 (figure 1, 3 et 4). Le réseau de réception 4 est quant à lui également un réseau d'antennes 4a régulièrement réparties, de préférence dans un plan, de manière à entourer au moins partiellement la cuve 2 (figure 2), voire selon un anneau qui entoure entièrement la cuve 2 (figures 1, 3 et 4).

Le réseau 3 d'antennes d'émission 3a et le réseau 4 d'antennes de réception 4a sont tous deux mobiles en translation le long de la cuve 2. Par ailleurs, le réseau de réception 4 est également mobile en rotation, selon un mouvement angulaire autour de la cuve 2 et du milieu à imager, notamment autour d'un axe colinéaire à la translation et de préférence dans son plan, qui permet le cas échéant de réaliser des sur-échantillonnages en réception. Le réseau 3 peut lui aussi être mobile en rotation autour de la cuve 2, notamment autour du même axe colinéaire à la translation que le réseau de réception 4 et de préférence dans son plan. Des motorisations (non représentées) sont prévues pour permettre les différents mouvements de rotation/translation souhaités.

Comme on l'aura compris, le réseau d'émission 3 et le réseau de réception 4 sont indépendants dans leurs mouvements et positionnements autour de la coque 2, ce qui permet un large choix possible de combinaisons d'émission et de réception et donc des traitements multistatiques complets et ainsi de meilleures performances d'imagerie, tout en autorisant un nombre d'antennes et une complexité et un coût de câblage limités. En particulier, et comme illustré, le réseau d'émission 3 et le réseau de réception 4 peuvent être mécaniquement indépendants à la fois dans leur mouvement de rotation angulaire et dans leur mouvement de translation.

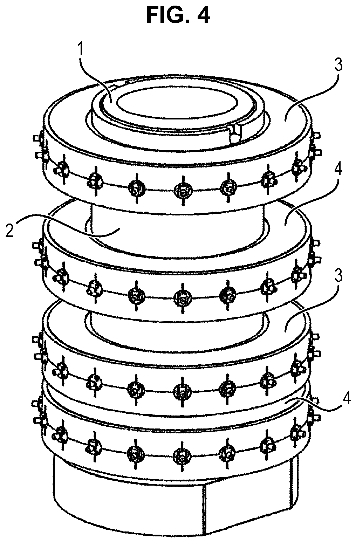

Plusieurs réseaux d'émission 3 et/ou plusieurs réseaux de réception 4 peuvent être prévus (voir figure 4). Ceci permet un choix possible de combinaisons d'émission et de réception plus large. Par exemple, dans le cas de deux réseaux d'émission 3 et de deux réseaux de réception 4, ceux-ci peuvent être empilés les uns sur les autres de manière à ce que les réseaux d'émission 3 alternent avec les réseaux de réception 4, ou que les réseaux d'émission 3 se situent de part et d'autre des réseaux de réception 4 et vice et versa.

Chaque réseau d'émission 3, en plus d'être mécaniquement indépendant des réseaux de réception 4, peut être mécaniquement indépendant des autres réseaux d'émission 3. De même, chaque réseau de réception 4, en plus d'être mécaniquement découplé des réseaux d'émission 3, peut être mécaniquement indépendant des autres réseaux de réception 4. Néanmoins, il est possible de rendre les réseaux d'émissions 3 mécaniquement dépendant les uns des autres, et les réseaux de réception 4 mécaniquement dépendant les uns des autres.

Dans le cas d'une pluralité de réseaux d'émission 3, et/ou de réseaux de réception 4, les réseaux d'émission 3 et de réception 4 peuvent être superposés et la course de translation de chacun des réseaux est limitée par les réseaux adjacents. Ceci permet de diminuer davantage les courses en translation des différents réseaux et ainsi de réduire la durée globale d'une acquisition complète autour de la cuve. Par exemple, chacun des réseaux 3, 4 est mobile en translation entre une première et deuxième positions extrémales : quand deux réseaux se touchent, l'un est dans sa première position extrémale et l'autre dans sa deuxième position extrémale. Ainsi, chacun des réseaux est mobile en translation sur une course dont la longueur est diminuée par rapport à la taille de la cuve 2.

Le ou les réseaux d'émission 3 et le ou les réseaux de réception 4 peuvent être bivalents. C'est-à-dire qu'ils peuvent être configurés de manière à agir autant en mode émission qu'en mode réception. Une commande est alors prévue pour la configuration des réseaux en mode émission, respectivement en mode réception. La commande est choisie de manière à contraindre la configuration des réseaux de telle sorte qu'au moins un réseau fonctionne en mode émission et au moins un réseau fonctionne en mode réception.

Le nombre d'antennes 3a du réseau 3 d'émission est choisi en fonction du nombre de points d'illumination désirés. Pour chaque point d'illumination, le signal est capté successivement par toutes les sondes de réception, en chaque point de la grille de « scan », c'est à dire de balayage, associant un déplacement mécanique du réseau de réception (translation verticale et rotation) et un balayage électronique des sondes de réception. On peut ainsi exploiter le caractère multistatique du trajet des ondes et augmenter la qualité du contraste et de la résolution des images reconstruites.

Les antennes 3a du réseau d'émission 3 sont par exemple des antennes bipolarisées, tandis que les antennes 4a du réseau 4 de réception sont des antennes mono-polarisées ou bipolarisées. Dans le cas où les réseaux d'émission 3 et de réception 4 sont bivalents, les antennes 3a, 4a de ces réseaux sont par exemple identiques mono-polarisées ou bipolarisées.

Les antennes sont par ailleurs avantageusement à large bande de façon à permettre l'émission et la réception de signaux radiofréquences sur une large bande de fréquences, ce qui permet d'augmenter la résolution et le contraste de l'image reconstruite. Par exemple, les fréquences d'émission/réception des antennes sont comprises entre 0.5 et 10 GHz.

Le dispositif peut également comprendre des éléments absorbants disposés entre deux antennes successives. Ceci permet d'isoler électro-magnétiquement les antennes entre elles.

Un système électronique permet la commande des déplacements des réseaux 3 et 4 (commande des motorisations afin de réaliser des balayages mécaniques), la gestion des capteurs mécaniques de butée de mouvement ou des capteurs de présence du sein à analyser, le multiplexage des sondes 3a, 4a et le traitement du signal radiofréquence.

Pour un exemple de traitement et d'analyse des signaux de réception permettant l'obtention d'images tomographiques 2D ou 3D, on pourra par exemple se référer aux publications suivantes :
- Y. Xie, B. Guo, L. Xu, J. Li, and P. Stoica, "Multistatic adaptative microwave imaging for early breast cancer detection", IEEE Trans. Biomed. Eng. , vol 53, n°8, pp 1647-1657, Aug. 2006 ; et
- R. Nilavalan, A. Gbedemah, I.J. Craddock, X. Li and S.C. Hagness, Numerical investigation of breast tumour detection using multi-static radar, ELECTRONICS LETTERS vol 39, n°25, December 2003.

Les antennes 3a du réseau 3, comme celles du réseau 4, sont directement moulées ou insérées dans un matériau de permittivité diélectrique identique à celle de la coque. De préférence ce matériau supportant les sondes possède des propriétés électromagnétiquement absorbantes de façon à réduire les couplages entre les sondes. En l'occurrence, dans le cas de l'application aux systèmes d'imagerie du sein, le matériau dans lequel les antennes sont moulées ou insérées est en anneau. D'autres formes de réseaux sont bien entendu possibles : réseaux linéaires ou en arcs de cercle non fermés, par exemple pour des systèmes d'imagerie de la thyroïde ou du genou.

La cuve 2 est également constituée d'un matériau de permittivité diélectrique identique à celle de la coque 1. Ce matériau constituant la cuve peut dans une autre réalisation posséder des propriétés électromagnétiquement absorbantes.

Le matériau de la coque 1 et de la cuve 2 est un matériau diélectrique à faibles pertes par exemple de l'Eccostock Hik500F de la société Emerson & Cuming.

En son intérieur, la coque 1 est elle-même recouverte d'un matériau biocompatible tel que du Nuflon (TGBBT).

Le matériau constituant les supports annulaires des sondes des réseaux 3 et 4 est un matériau possédant à la fois la même permittivité diélectrique que celle de l'Eccostock Hik500F et une capacité d'absorption des ondes électromagnétiques.

Dans une autre réalisation illustrée à la Figure 2, les supports - référencés par 3b et 4b - des sondes des réseaux 3 et 4 peuvent être constitués d'anneaux en matériau diélectrique à faibles pertes, par exemple de l'Eccostock Hik500F, contenant des inserts 10 entre les sondes réalisés en matériau possédant à la fois la même permittivité diélectrique que celle de l'Eccostock Hik500F et une capacité d'absorption des ondes électromagnétiques. Le support 3b qui porte les sondes 3a d'émission est monté sur des vérins 11 qui permettent le déplacement en translation dudit support 3b. Ces vérins 11 sont eux-mêmes montés sur un rail annulaire 3c, qui permet au support 3b de tourner autour de l'axe de la coque 1. Le support 4b qui porte les sondes 4a de réception est monté sur des vérins 12 qui permettent le déplacement en translation dudit support 4b. Ces vérins 12 sont eux-mêmes montés sur un rail annulaire 4c, qui permet au support 4b de tourner autour de l'axe de la coque 1.

Le matériau absorbant solide est par exemple réalisé à base de silicone ou d'époxy ayant la même permittivité diélectrique réelle que le matériau constituant la coque 1. Ce matériau est en outre chargé de poudre absorbante par exemple des charges ferromagnétiques, de façon à limiter le couplage direct entre les antennes. Un tel matériau absorbant permet une atténuation du champ électromagnétique d'environ 4dB/cm à 1GHz, 12dB/cm à 3GHz, 18dB/cm à 6GHz et 18.5dB/cm à 8 GHz. On notera qu'une telle solution a l'avantage de permettre d'obtenir pour le dispositif un ensemble totalement intégré sans élément absorbant en mousse qu'il serait nécessaire d'interposer entre les sondes et qui poserait, du point de vue de l'hygiène (présence de particules de carbone volantes), des problématiques importantes.

Par ailleurs, le fait d'utiliser des matériaux de mêmes constantes diélectriques pour la coque 1, la cuve 2 et les supports des antennes des réseaux 3 et 4 permet d'éviter l'apparition d'échos parasites produits par la réflexion aux interfaces. En outre, le fait que les antennes d'émission soient rigidement liées ensemble dans un même support permet d'éviter les mouvements relatifs et permet un maintien excellent de la stabilité des phases des signaux mesurés et donc des résultats d'imagerie améliorés.

Par ailleurs, pour compléter l'isolation des sondes entre elles, il est également possible de prévoir des commutateurs radiofréquences avec des caractéristiques d'isolation élevées.

On prévoit également en outre un marquage à la réception des sondes, ainsi que le cas échéant à l'émission des sondes, par modulation suivant la technique de la diffusion modulée qui permet de marquer localement le champ à l'endroit de la réception, ainsi que le cas échéant à l'émission.

Ceci permet de réduire la complexité et les coûts de l'électronique en s'affranchissant de matrices de commutateurs entre sondes complexes et onéreuses.

Lorsque les antennes d'émission et de réception sont bipolarisées, l'électronique de commande (non représentée) sélectionne la polarisation des antennes de travail (soit au moyen des commutateurs, soit par modulation, soit même en combinant les deux).

Les carters métalliques 5 forment des cages de Faraday partielles.

Ils sont associés à des matériaux absorbants par exemple en mousse souple ou rigide 6 disposés :
- d'une part entre les deux carters métalliques 5 et en particulier sur la face intérieure du carter 5 extérieur ;
- d'autre part entre les réseaux 3 et 4 d'antennes et lesdits carters.

Ces matériaux permettent d'atténuer fortement, voire d'éliminer, les échos du signal électromagnétique et ainsi d'éviter tout artefact de reconstruction d'image qui serait dû à des réflexions multiples.

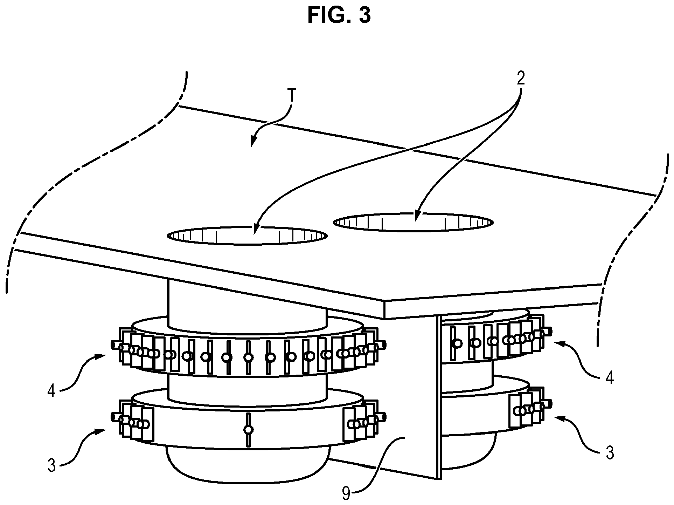

Comme illustré sur la figure 3 pour un autre mode de réalisation, le système d'imagerie peut également comprendre deux cuves juxtaposées 2, chacune associée à un sous-système de réseaux d'émission/réception 3 et 4.

Une plaque métallique 9 recouverte d'absorbant isolant électromagnétiquement les deux cuves est alors interposée entre les deux sous-systèmes.

De cette façon, on diminue par deux la durée de l'examen et on présente simultanément et en parallèle l'évolution de l'image obtenue et reconstruite, ce qui permet un examen et un diagnostic par comparaison des deux seins.

Par ailleurs, en fonction des zones du milieu sous observation à imager, il peut être intéressant d'incliner le système d'émission et de réception, par rapport au plan de la table T sur laquelle la patiente vient se positionner, par exemple un mécanisme d'inclinaison peut être prévu pour commander l'inclinaison des plans des réseaux symétriquement par rapport à la plaque métallique 9 et de manière à ce que les antennes 3a, 4a des réseaux les plus proche de la plaque métallique 9 s'éloignent de la patiente, c'est-à-dire du plan de la table T, et les antennes 3a, 4a des réseaux les plus éloignées de la plaque métallique 9 se rapprochent de la patiente, c'est-à-dire du plan de la table T. L'angle d'inclinaison des réseaux par rapport au plan T de la table est par exemple de l'ordre de 15 degrés.

Dans le cas de l'imagerie du sein, cette inclinaison des plans des réseaux peut être utile pour scanner les zones extérieures du sein, et en particulier la zone de prolongement axillaire de la glande mammaire.

Dans un mode de réalisation, le système d'imagerie médicale comprend :
- un réseau 3 d'antennes 3a d'émission et un réseau 4 d'antennes 4a de réception, et
- une cuve 2 configurée pour recevoir une coque 1 destinée à recevoir le milieu de tissu humain.

Un procédé d'imagerie médicale d'un milieu de tissu humain, comme par exemple le sein d'une patiente, consiste à déplacer au moins l'un de ces réseaux par rapport au volume sous observation recevant le milieu de tissu humain, notamment en rotation angulaire et/ou en translation, comme mentionné précédemment. Il est possible de déplacer les réseaux 3, 4 en rotation angulaire et en translation de manière indépendante l'un de l'autre.

Dans un mode de réalisation possible, les micro-ondes émises par le réseau d'émission et arrivant à la coque 1 traversent un milieu de transition uniquement solide. Ce milieu de transition uniquement solide est constitué par exemple par la cuve 2 (comme en Figure 1), ou par le support 3b et la cuve 2 (comme en Figure 2). Ceci permet de s'affranchir des inconvénients des milieux de transition liquides.

On note que selon les applications, il n'est toutefois pas obligatoire d'utiliser un milieu de transition uniquement solide.

Dans un exemple particulier, les micro-ondes émises par le réseau 3 d'émission et arrivant au milieu de tissu humain à observer (comme le sein de la patiente) traversent un milieu de transition uniquement solide.

Ce milieu de transition uniquement solide est constitué par exemple par la cuve 2 et la coque 1 (comme en Figure 1), ou par le support 3b, la cuve 2 et la coque 1 (comme en Figure 2).

Ainsi, dans cet exemple non limitatif, aucun milieu de transition liquide n'est utilisé entre le réseau 3 d'émission et le milieu de tissu humain à observer.

L'invention trouve notamment une application à l'imagerie du sein.

## Revendications

1. Système d'imagerie médicale à antennes d'émission micro-ondes et antennes de réception du champ électromagnétique disposées autour d'un volume destiné à recevoir un milieu de tissu humain à observer, comportant:
- un réseau (3) d'antennes (3a) d'émission et un réseau (4) d'antennes (4a) de réception, ces deux réseaux (3, 4) étant mécaniquement indépendants,
- des motorisations configurées pour déplacer le réseau (3) d'émission et le réseau (4) de réception en rotation angulaire et en translation par rapport au volume sous observation, afin de permettre un balayage de celui-ci, et
- une coque (1) en matériau diélectrique solide définissant le volume destiné à recevoir un milieu de tissu humain à observer, les antennes (3a) d'émission et les antennes (4a) de réception étant disposées en réseau dans des supports (3b, 4b), la coque (1) étant disposée dans une cuve (2) en un matériau diélectrique que les réseaux (3, 4) entourent au moins partiellement, les matériaux de la cuve (2), de la coque (1) et des supports (3b, 4b) de réseaux possédant des permittivités diélectriques identiques.

2. Système d'imagerie selon la revendication 1, caractérisé en ce les deux réseaux (3, 4) sont indépendants en rotation et en translation.

3. Système selon la revendication 1 ou la revendication 2, **caractérisé en ce que** lesdits supports (3b, 4b) comportent des parties en un matériau électromagnétiquement absorbants entre les antennes (3a, 4a).

4. Système selon la revendication 1 ou la revendication 2, **caractérisé en ce que** les supports (3b, 4b) sont constitués entièrement en un matériau électromagnétiquement absorbant.

5. Système selon l'une des revendications 3 à 4, **caractérisé en ce que** la coque (1) est disposée dans une cuve (2) en un matériau diélectrique que les réseaux (3, 4) entourent au moins partiellement, les matériaux de la cuve (2), de la coque (1) et des supports (3b, 4b) de réseaux étant identiques.

6. Système selon la revendication 5, **caractérisé en ce que** la cuve (2) est extérieurement cylindrique, le réseau (3) d'émission et/ou le réseau (4) de réception se déplaçant en translation et/ou rotation autour de celle-ci.

7. Système selon la revendication 1 ou la revendication 2, **caractérisé en ce qu'**il comporte au moins deux cuves (2).

8. Système selon l'une des revendications 4 à 5, **caractérisé en ce que** la coque (1) est amovible, le système étant associé à un jeu de coques de dimensions internes différentes.

9. Système selon l'une des revendications 1 à 8, **caractérisé en ce qu'**il comprend une électronique qui met en oeuvre une différenciation au moins des antennes (3a) de réception par modulation.

10. Système selon l'une des revendications 1 à 9, **caractérisé en ce qu'**il comprend au moins deux réseaux (3) d'antennes (3a) de réception et/ou au moins deux réseaux (4) d'antennes (4a) d'émission.

11. Système selon la revendication 10, **caractérisé en ce que** chacun des réseaux (3, 4) d'antennes est mobile en translation de manière limitée.

12. Système selon l'une des revendications 1 à 11, **caractérisé en ce que** le ou les réseaux (3) d'antennes (3a) de réception et le ou les réseaux (4) d'antennes (4a) d'émission sont bivalents, et **en ce qu'**il comprend en outre une commande pour la configuration des réseaux (3, 4) en mode émission, respectivement en mode réception, la commande est choisie de manière à contraindre la configuration des réseaux (3, 4) de telle sorte qu'au moins un réseau fonctionne en mode émission et au moins un réseau fonctionne en mode réception.

13. Procédé d'imagerie médicale d'un milieu de tissu humain à observer, dans un système d'imagerie médicale selon la revendication 1, le procédé comprenant l'étape consistant à déplacer au moins l'un de ces réseaux (3, 4) en rotation angulaire et en translation par rapport au volume sous observation, afin de permettre un balayage de celui-ci, les micro-ondes émises par le réseau (3) d'émission et arrivant à la coque (1) traversant un milieu de transition uniquement solide.

## Patentansprüche

1. Medizinisches Bildgebungssystem mit Mikrowellen-Sendeantennen und Antennen für den Empfang des elektromagnetischen Feldes, die um ein Volumen herum angeordnet sind, das dazu bestimmt ist, ein zu beobachtendes menschliches Gewebemedium aufzunehmen, umfassend:
- ein Array (3) aus Sendeantennen (3a) und ein Array (4) aus Empfangsantennen (4a), wobei diese beiden Arrays (3, 4) mechanisch unabhängig sind,
- Motorisierungen, die dazu konfiguriert sind, das Sendearray (3) und das Empfangsarray (4) in einer Winkeldrehung und in Translation in Bezug auf das Beobachtungsvolumen zu bewegen, um die Abtastung des letzteren zu ermöglichen, und
- eine Schale (1) aus festem dielektrischem Material, die das Volumen definiert, das dazu bestimmt ist, das zu beobachtende menschliche Gewebemedium aufzunehmen, wobei die Sendeantennen (3a) und die Empfangsantennen (4a) in einem Array von Trägern (3b, 4b) angeordnet sind, wobei die Schale (1) in einem Behälter (2) aus einem dielektrischen Material angeordnet ist, den die Arrays (3, 4) zumindest teilweise umgeben, wobei die Materialien des Behälters (2), der Schale (1) und der Träger (3b, 4b) der Arrays über identische Dielektrizitätskonstante verfügen.

2. Bildgebungssystem nach Anspruch 1, **dadurch gekennzeichnet, dass** die beiden Arrays (3, 4) in Rotation und Translation unabhängig sind.

3. System nach Anspruch 1 oder Anspruch 2, **dadurch gekennzeichnet, dass** die Träger (3b, 4b) Teile aus einem elektromagnetisch absorbierenden Material zwischen den Antennen (3a, 4a) umfassen.

4. System nach Anspruch 1 oder Anspruch 2, **dadurch gekennzeichnet, dass** die Träger (3b, 4b) vollständig aus einem elektromagnetisch absorbierenden Material bestehen.

5. System nach einem der Ansprüche 3 bis 4, **dadurch gekennzeichnet, dass** die Schale (1) in einem Behälter (2) aus einem dielektrischen Material angeordnet ist, der die Arrays (3, 4) zumindest teilweise umgibt, wobei die Materialien des Behälters (2), der Schale (1) und der Träger (3b, 4b) der Arrays identisch sind.

6. System nach Anspruch 5, **dadurch gekennzeichnet, dass** der Behälter (2) außen zylindrisch ist, wobei sich das Sendearray (3) und/oder das Empfangsarray (4) translatorisch und/oder rotierend um letzteres bewegen.

7. System nach Anspruch 1 oder Anspruch 2, **dadurch gekennzeichnet, dass** es mindestens zwei Behälter (2) aufweist.

8. System nach einem der Ansprüche 4 bis 5, **dadurch gekennzeichnet, dass** die Schale (1) abnehmbar ist, wobei das System mit einem Satz Schalen mit unterschiedlichen Innenabmessungen verbunden ist.

9. System nach einem der Ansprüche 1 bis 8, **dadurch gekennzeichnet, dass** es eine Elektronik umfasst, die durch Modulation eine Differenzierung zumindest der Empfangsantennen (3a) durchführt.

10. System nach einem der Ansprüche 1 bis 9, **dadurch gekennzeichnet, dass** es mindestens zwei Arrays (3) von Empfangsantennen (3a) und/oder mindestens zwei Arrays (4) von Sendeantennen (4a) umfasst.

11. System nach Anspruch 10, **dadurch gekennzeichnet, dass** jedes der Arrays (3, 4) von Antennen in begrenzter Weise translatorisch beweglich ist.

12. System nach einem der Ansprüche 1 bis 11, **dadurch gekennzeichnet, dass** das oder die Arrays (3) von Empfangsantennen (3a) und das oder die Arrays (4) von Sendeantennen (4a) bivalent sind, und dadurch, dass es ferner einen Befehl für die Konfiguration der Arrays (3, 4) im Sendebetrieb bzw. im Empfangsmodus umfasst, wobei der Befehl jeweils so gewählt wird, dass die Konfiguration der Arrays (3, 4) so erzwungen wird, dass mindestens ein Array im Sendemodus und mindestens ein Array im Empfangsmodus betrieben wird.

13. Verfahren zur medizinischen Bildgebung eines zu beobachtenden menschlichen Gewebemediums in einem medizinischen Bildgebungssystem nach Anspruch 1, wobei das Verfahren mindestens den Schritt umfasst, der darin besteht, zumindest eines dieser Arrays (3, 4) in Winkeldrehung und in Translation in Bezug auf das Beobachtungsvolumen zu verschieben, um die Abtastung des letzteren zu ermöglichen, wobei die Mikrowelle, die vom Sendearray (3) ausgesendet und an der Schale (1) ankommen, ein einziges festes Übergangsmedium durchqueren.

## Claims

1. Medical imaging system with micro-wave emission antennae and electromagnetic field reception antennae arranged around a volume intended to receive a human tissue to be observed, comprising :
- a network (3) of transmitting antennae (3a) and a network (4) of receiving antennae (4a), these two arrays (3, 4) being mechanically independent,
- motorisations configured to move the emitting network (3) and the receiving network (4) in angular rotation and in translation relative to the volume under observation, so as to enable it to be scanned, and
- a shell (1) of solid dielectric material defining the volume intended to receive a medium of human tissue to be observed, the transmitting antennae (3a) and the receiving antennae (4a) being arranged in a network in supports (3b, 4b), the shell (1) being arranged in a tank (2) made of a dielectric material which the networks (3a, 4b) surround at least partially the materials of the tank (2), the shell (1) and the supports (3b, 4b) of networks having identical dielectric permittivities.

2. Imaging system as claimed in claim 1, **characterised in that** the two networks (3, 4) are independent in rotation and in translation.

3. System according to Claim 1 or Claim 2, **characterised in that** the said supports (3b, 4b) comprise parts made of an electromagnetically absorbent material between the antennae (3a, 4a).

4. System according to Claim 1 or Claim 2, **characterised in that** the supports (3b, 4b) are made entirely of an electromagnetically absorbent material.

5. System according to one of claims 3 to 4, **characterised in that** the shell (1) is arranged in a tank (2) made of a dielectric material which the networks (3, 4) surround at least partially, the materials of the tank (2), of the shell (1) and of the supports (3b, 4b) of the networks being identical.

6. System according to claim 5, **characterised in that** the tank (2) is externally cylindrical, with the transmission network (3) and/or the reception network (4) moving in translation and/or in rotation around it.

7. System according to claim 1 or claim 2, **characterised in that** it comprises at least two tanks (2).

8. System according to one of claims 4 to 5, **characterised in that** the shell (1) is removable, the system being associated with a set of shells of different internal dimensions.

9. System according to one of claims 1 to 8, **characterised in that** it comprises electronics which implement a differentiation of at least the reception antennas (3a) by modulation.

10. System according to one of claims 1 to 9, **characterised in that** it comprises at least two networks (3) of receiving antennas (3a) and/or at least two networks (4) of transmitting antennas (4a).

11. System according to claim 10, **characterised in that** each of the antennae networks (3, 4) is mobile in translation to a limited extent.

12. System according to one of claims 1 to 11, **characterised in that** the network(s) (3) of receiving antennae (3a) and the network(s) (4) of transmitting antennae (4a) are bivalent, and **in that** it further comprises a control for configuring the networks (3, 4) in transmission mode, respectively in reception mode, the control is chosen so as to constrain the configuration of the networks (3, 4) in such a way that at least one network operates in transmission mode and at least one network operates in receiving mode.

13. Method for medical imaging of a human tissue medium to be observed, in a medical imaging system according to claim 1, the method comprising the step consisting in moving at least one of these networks (3, 4) in angular rotation and in translation relative to the volume under observation, so as to enable it to be scanned, the microwaves emitted by the emission network (3) and arriving at the shell (1) going through a purely solid transition medium.
